# EUROPEAN PATENT SPECIFICATION

(11) **EP 2 231 211 B1**
(45) Date of publication and mention of the grant of the patent: **24.10.2012**
(21) Application number: 08860438.4
(22) Date of filing: 12.12.2008
(51) Int. Cl.: A61L 29/08, A61L 29/14, A61L 31/10, A61L 31/14

(54) **MEDICAL DEVICE COATINGS AND METHODS OF FORMING SUCH COATINGS**
BESCHICHTUNGEN VON MEDIZINPRODUKTEN UND VERFAHREN ZUR BILDUNG SOLCHER BESCHICHTUNGEN
REVÊTEMENTS DE DISPOSITIF MÉDICAL ET PROCÉDÉS DE FORMATION DE CES REVÊTEMENTS

(30) Priority: 13.12.2007 US 956223
(43) Date of publication of application: 29.09.2010
(73) Proprietor: Boston Scientific Scimed, Inc., Maple Grove, MN 55311 (US)
(72) Inventor: LIN, Horng-Ban, Maple Grove MN 55311 (US); KANGAS, Steve, Woodbury MN 55125 (US); JOHNSON, David, K., Brooklyn Park MN 55443 (US)
(74) Representative: Vossius & Partner
(86) International application number: PCT/US2008/086685
(87) International publication number: WO 2009/076639

(56) References cited:
- WO-A1-2006/037321
- WO-A1-2006//037321
- WO-A2-2008/055718
- US-A1- 2002 013 549
- US-A1- 2006 095 067
- US-A1- 2007 014 945

## Description

### Technical Field

This disclosure pertains to coatings on medical devices and methods for applying coatings to medical devices.

### Background

Coatings can be applied to objects such as medical devices for many different purposes. For example, coatings can provide desired surface properties such as high or low lubricity, hydrophilicity, hydrophobicity, biocompatibility, or other properties, or combinations thereof. The durability of the coating and maintaining the coating properties before and during use can also be important. There is an ongoing need to provide different medical device coatings and different methods of applying coatings to medical devices.

### Summary

The invention provides several alternative coatings, including alternative coating compositions, and methods of applying these coatings to a variety of surfaces.

Accordingly, in one embodiment a medical device comprises an elongated member with a surface. A coating having a first layer and a second layer is disposed on the medical device. The first layer is disposed on at least a portion of the surface of the medical device. This first layer comprises first and second polymeric units. The first polymeric unit adheres to the surface of the medical device, and the second polymeric unit comprises polyurethane. The second layer is disposed on at least a portion of the first layer, and the second layer comprises third and fourth polymeric units. The third polymeric unit comprises polyurethane and the fourth polymeric unit provides the surface properties of the coating. The fourth polymeric unit is hydrophilic.

In some embodiments the second and third polymeric units can be the same polyurethane polymeric unit. Further, the first polymeric unit including a polyester and the fourth polymeric unit could be a variety of polymeric units including poly (ethylene oxide), poly (propylene oxide), polyether, poly (ethylene glycol) and poly (propylene glycol).

In another embodiment a method of coating a medical device is disclosed. The medical device comprises an elongated member and the elongated member has a surface. The coating has a first and a second layer. The first layer is applied to at least a portion of the surface of the medical device. The first layer as defined in claim 14 can be applied in the form of a solution or suspension or dispersion or other liquid medium that comprises first and second polymer units, for example, as discussed herein. In some embodiments, the first layer can be dried after it is applied. Further, the second layer is applied to at least a portion of the first layer. The second layer as defined in claim 14 can be applied in the form of a solution or suspension or dispersion or other liquid medium that comprises third and fourth polymeric units, for example, as discussed herein. In some embodiments, the second layer can be dried after it is applied. The first and second layers can be applied using a variety of processes including a spray coating process, a dip coating process, or a contact-type process such as brushing, wiping or rolling the liquid on the surface.

The Figures, and Detailed Description which follows, more particularly exemplify these embodiments.

### Brief Description of the Figures

The invention may be more completely understood in consideration of the following detailed description of various embodiments of the invention in connection with the accompanying drawings, in which:
Figure 1 shows a cross-section of a coated surface of a medical device;
Figure 2 shows a cross-section of a portion of a coated guidewire;
Figure 3 shows a cross-section of a portion of a coated catheter;
Figure 4 shows a surface of a medical device with a first layer of a coating being applied;
Figure 5 shows a surface of a medical device with a first layer applied;
Figure 6 shows the medical device of Figure 5 with a second layer of a coating being applied;
Figure 7 shows the medical device of Figure 5 with a second layer of a coating applied; and
Figure 8 shows a chart of the lubricity of several samples over a number of wear cycles.

### Detailed Description

The following description should be read with reference to the drawings wherein like reference numerals indicate like elements throughout the several views. The drawings, which are not necessarily to scale, depict illustrative embodiments of the claimed invention.

Medical device coatings can be used for a variety of purposes. For example, a coating can be applied to a surface or surfaces of a medical device in order to give the surface or surfaces certain properties. Coatings can provide a surface with a high or low lubricity, the coating can be hydrophobic or hydrophilic (e.g., it could be a hydrogel) or biocompatible or have other properties, or the coating could have a combination of such properties. In the case of some hydrogel coatings, the hydrogel can provide a surface with increased lubricity when the hydrogel has absorbed water. In some cases, such hydrogel coatings can be useful in applications in which a medical device will be placed inside a patient's body, for example when the medical device may come into contact with blood, such as in the vasculature of a patient.

In addition, the durability of the coating can be important to the overall performance of the medical device. For example, a more durable coating can allow the properties of the coating to be more effectively maintained before and during use.

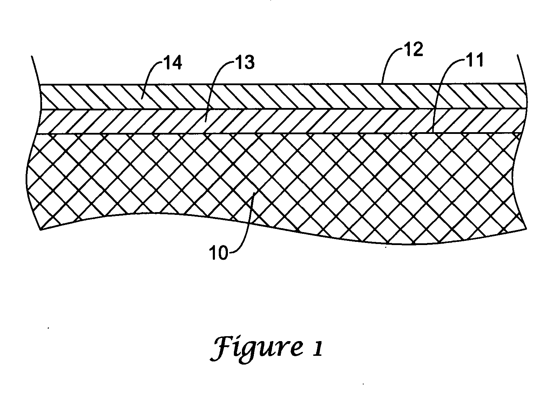

Turning to Figure 1, the surface of a medical device 10 is shown with a coating 12 disposed on a surface 11 of the medical device 10. In this example, the coating 12 can comprise a first layer 13 and a second layer 14. The first layer 13 can be disposed on at least a portion of the surface 11 of the medical device 10. The first layer 13 can also be disposed on substantially all, or on the entire, surface of the medical device 10. In this example, the surface that is shown is an outside exposed surface of the medical device 10, although it is also contemplated that the coatings described herein can be disposed on an inside surface, or any other surface, of a medical device. Further, coverage of the coated portion of the surface by the first layer can vary from covering the entire coated portion, substantially all of the coated portion, or the first layer can cover at least 50%, at least 60%, at least 70% or at least 80% of the coated portion of the surface.

The first layer 13 comprises first and second functionalities. The first functionality adheres to the surface 11 of the medical device 10. The second functionality adheres to the second layer 14, as discussed below. The first and second functionalities can both be present as polymers and/or polymeric units within the first layer. In some embodiments, the first and second functionalities and/or polymer units can be considered to form and/of be a copolymer, such as a random, graft, or block copolymer, and the copolymer may form at least a part of, if not substantially all of, the first layer 13. In some other embodiments, the first and second functionalities and/or polymer units may not be considered to form a copolymer, but rather a mixture or dispersion that forms the first layer 13. For example, the first layer may comprise one of the first or second polymer units (e.g. polymers) dispersed within the other of the first or second polymer units (e.g. polymers) to form a dispersion. In some embodiments, combining these functionalities in one compound can simplify the first layer; for example, having fewer components (e.g., one copolymer rather than two polymers) in the first layer 13 can in some cases lead to a first layer that is easier to make and/or apply.

Further, in some embodiments, the first layer 13 can consist essentially of the first and second functionalities and/or polymer units. For example, in some embodiments, the first layer 13 (for example, when cured) can consist essentially of a copolymer of the first and second functionalities and/or polymer units. As another example, in some embodiments, the first layer 13 (for example, when cured) can be a dispersion that consists essentially of the first and second functionalities and/or polymer units. In other cases, the first layer 13 can also comprise other materials such as radiopaque materials or materials that affect the MRI compatibility of the first layer 13.

The second layer 14 is disposed on at least a portion of the first layer 13. For example, the second layer 14 can be disposed on substantially all, or on the entire, first layer 13. In some embodiments, portions of the second layer 14 can extend beyond the first layer 13 and at least a portion of the second layer 14 can be disposed on at least a portion of a surface 11 of the medical device 10. In other embodiments the second layer 14 can cover only a portion of the first layer 13, and in some cases at least a portion of the first layer 13 can remain exposed.

The second layer 14 comprises third and fourth functionalities. The third functionality adheres to the first layer 13. The second functionality of the first layer and the third functionality of the second layer adhere to one another. The fourth functionality can provide for certain surface properties on the medical device 10. For example, the fourth functionality provides a hydrophilic surface.

The third and fourth functionalities can both be present as polymers and/or polymer units within the second layer.

In some embodiments, the third and fourth functionalities and/or polymer units can be considered to form and/or be a copolymer, such as a random, graft, or block copolymer, and the copolymer may form at least a part of, if not substantially all of, the second layer 14. In some other embodiments, the third and fourth functionalities and/or polymer units may not be considered to form a copolymer, but rather a mixture or dispersion that forms the second layer 14. For example, the second layer may comprise one of the third or fourth polymer units (e.g. polymers) dispersed within the other of the third and fourth polymer units (e.g. polymers) to form a dispersion. In some embodiments, combining these functionalities in one compound can simplify the second layer; for example, having fewer components (e.g., one copolymer rather than two polymers) in the second layer 14 can in some cases lead to a second layer that is easier to make and/or apply.

Further, in some embodiments, the second layer 14 can consist essentially of the third and fourth functionalities and/or polymer units. For example, in some embodiments, the second layer 14 (for example, when cured) can consist essentially of a copolymer of the third and fourth functionalities and/or polymer units. As another example, in some embodiments, the second layer 14 (for example, when cured) can be a dispersion that consists essentially of the third and fourth functionalities and/or polymer units. In other cases, the second layer 14 can also comprise other materials such as radiopaque materials or materials that affect the MRI compatibility of the second layer 14.

As mentioned above, the first functionality can adhere to the surface 11 of the medical device 10. The medical device 10, or portions thereof, including the surface 11, can comprise a variety of materials including polymers, ceramic materials, metallic materials, or other suitable materials. Possible polymeric materials include PTFE, fluorinated ethylene propylene (FEP), polyurethane, polypropylene (PP), polyvinylchloride (PVC), polyether-ester (for example a polyether-ester elastomer such as ARNITEL® available from DSM Engineering Plastics), polyester (for example a polyester elastomer such as HYTREL® available from DuPont), polyamide (for example, DURETHAN® available from Bayer or CRISTAMID® available from Elf Atochem), elastomeric polyamides, block polyamide/ethers, polyether block amide (PEBA, for example available under the trade name PEBAX®), silicones, polyethylene, Marlex high-density polyethylene, linear low density polyethylene (for example REXELL®), polyetheretherketone (PEEK), polyimide (PI), polyetherimide (PEI), other suitable materials, or mixtures, combinations, or copolymers thereof. In some embodiments materials or the medical device and/or surface 11 thereof, can include a liquid crystal polymer (LCP) blended with other polymers to enhance torqueability.

In addition, some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL® 625, UNS: N06022 such as HASTELLOY® C-22®, UNS: N10276 such as HASTELLOY® C276®, other HASTELLOY® alloys, and the like), nickel-copper alloys (e.g., UNS: N04400 such as MONEL® 400, NICKELVAC® 400, NICORROS® 400, and the like), nickel-cobalt-chromium-molybdenum alloys (e.g., UNS: R30035 such as MP35-N® and the like), nickel-molybdenum alloys (e.g., UNS: N10665 such as HASTELLOY® ALLOY B2®), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY®, PHYNOX®, and the like); platinum enriched stainless steel; combinations thereof; and the like; or any other suitable material.

The word Nitinol was coined by a group of researchers at the United States Naval Ordinance Laboratory (NOL) who were the first to observe the shape memory behavior of this material. The word Nitinol is an acronym including the chemical symbol for nickel (Ni), the chemical symbol for titanium (Ti), and an acronym identifying the Naval Ordinance Laboratory (NOL). In some embodiments, Nitinol alloys can include in the range of about 50 to about 60 weight percent nickel, with the remainder being essentially titanium. It should be understood, however, that in other embodiment, the range of weight percent nickel and titanium, and/or other trace elements may vary from these ranges. Within the family of commercially available Nitinol alloys, are categories designated as "superelastic" (i.e. pseudoelastic) and "linear elastic" which, although similar in chemistry, exhibit distinct and useful mechanical properties.

In some embodiments, a superelastic alloy, for example a superelastic Nitinol can be used to achieve desired properties. Such alloys typically display a substantial "superelastic plateau" or "flag region" in its stress/strain curve. Such alloys can be desirable in some embodiments because a suitable superelastic alloy can provide structure that exhibits some enhanced ability, relative to some other non-superelastic materials, of substantially recovering its shape without significant plastic deformation, upon the application and release of stress, for example, during insertion or navigation of the guidewire in the body.

In some other embodiments, a linear elastic alloy, for example a linear elastic Nitinol can be used to achieve desired properties. For example, in some embodiments, certain linear elastic Nitinol alloys can be generated by the application of cold work, directional stress, and heat treatment, such that the material fabricated does not display a substantial "superelastic plateau" or "flag region" in its stress/strain curve. Instead, in such embodiments, as recoverable strain increases, the stress continues to increase in a somewhat linear relationship until plastic deformation begins. In some embodiments, the linear elastic nickel-titanium alloy can be an alloy that does not show any martensite/austenite phase changes that are detectable by DSC and DMTA analysis over a large temperature range. For example, in some embodiments, there may be no martensite/austenite phase changes detectable by DSC and DMTA analysis in the range of about -60°C to about 120°C, and in other embodiments, in the range of about -100°C to about 100°C. The mechanical bending properties of such material are therefore generally inert to the effect of temperature over a broad range of temperature. In some particular embodiments, the mechanical properties of the alloy at ambient or room temperature are substantially the same as the mechanical properties at body temperature. In some embodiments, the use of the linear elastic nickel-titanium alloy can provide for structure that exhibits superior "pushability" around tortuous anatomy. One example of a suitable nickel-titanium alloy exhibiting at least some linear elastic properties is FHP-NT alloy commercially available from Furukawa Techno Material Co. of Kanagawa, Japan. Additionally, some examples of suitable nickel-titanium alloy exhibiting at least some linear elastic properties include those disclosed in U.S. Patent Nos. 5,238,004 and 6,508,803.

In some cases, the surface 11 of the medical device 10 can comprise a material to which some coatings do not normally adhere strongly. For example, some polyurethane based coatings do not adhere strongly to certain metallic surfaces such as stainless steel or Nitinol or to ceramic surfaces. In such cases, the first functionality of the first layer described herein can adhere to the surface 11, allowing for improved adhesion of the coating to the surface 11 relative to the adhesion of a coating without the first functionality present in the first layer. As such, in the context of this patent application, when it is stated that a functionality or a polymeric unit adheres to a surface or to another portion of the coating, this means that the coating or the layer of the coating has improved adhesion compared to the level of adhesion if the functionality or polymeric unit were not present in the coating or layer. Without being bound by the theory, it is thought that the first functionalities (i.e., the polyester) have a greater affinity for metallic or ceramic surfaces than do the second functionalities (i.e., the polyurethane). As such, a first layer with both these first and second functionalities present can have improved adhesion to certain metallic or ceramic surfaces compared to a polymer of the second functionality alone.

Similarly, the second functionalities (i.e., the polyurethane) and the third functionalities (i.e., the polyurethane) have an affinity for one another. As such, first and second layers with such second and third functionalities, respectively, have improved adhesion to one another compared to first and second layers without the second and third functionalities present. In some cases, improved adhesion to the medical device surface and between layers can result in improved durability of the coating, which will be discussed in more detail with respect to the Examples included below.

In some cases, the adhesion between the first functionality and a surface of the medical device can be by ionic bonding or van der Walls bonding or intermolecular hydrogen bonding or other types of bonding that does not entail the sharing of electrons, or any combination of these types of bonds. Further, the adhesion of the first and second layers to one another can be by similar bonding mechanisms. In this respect, the interface between the first layer and the surface of the medical device and the interface between the two layers is without chemical bonding in which electrons are shared (e.g., cross-linking or covalent bonding).

As mentioned above, the first layer 13 of the coating in Figure 1 comprises first and second functionalities. The first functionality comprises a first polymeric unit and the second functionality comprises a second polymeric unit. The first polymeric unit is a polymeric unit that adheres to the surface 11 of the medical device 10. This first polymeric unit comprises a polyester. The second polymeric unit is a polymeric unit that adheres to the second layer. The second polymeric unit adheres to the third functionality of the second layer. This second polymeric unit comprises a polyurethane.

The second layer 14 of the coating in Figure 1 comprises a composition and/or copolymer with third and fourth functionalities. The third functionality comprises a third polymeric unit and the fourth functionality comprises a fourth polymeric unit. The third polymeric unit is a polymeric unit that adheres to the second polymeric unit of the first layer 13. This third polymeric unit comprises a polyurethane. In some cases, the third polymeric unit can comprise the same polymeric unit as the second polymeric unit, for example the same polyurethane as the second polymeric unit.

The fourth polymeric unit provides the surface properties of the coating. The fourth polymeric unit has hydrophilic properties. In some examples, the fourth polymeric unit can form a hydrogel. The hydrogel can provide for increased lubricity when water is absorbed into the hydrogel. In some cases, the hydrogel can absorb five times or more of its own weight in water. In other cases, the hydrogel can absorb twenty times or more its own weight in water.

In some embodiments, the arrangement of a first layer and a second layer as discussed herein can allow for the separation of functionalities. In other words, the first functionality in the first layer (e.g., the polymeric unit that adheres to the surface of the medical device) can be in close proximity to the surface of the medical device. Further, the fourth functionality in the second layer (e.g., the polymeric unit that provides for the desired surface properties of the coating) can be in close proximity to a surface of the coating that is exposed to the surrounding environment. In this way, the effectiveness of the functionalities can be enhanced by maintaining them apart from one another and/or by disposing higher concentrations of the functionalities where they are most useful.

Further, the polymeric units described herein can comprise single or multiple chemical moieties; in some cases, the polymeric units can have multiple moieties that together make up the polymeric unit. For example, the first polymeric unit can have an ester moiety along with one or more other moieties. The second polymeric units described herein can also have multiple moieties, such as a urethane moiety along with one or more other moieties. The third polymeric units described herein can also have multiple moieties such as a urethane moiety along with one or more other moieties. The fourth polymeric unit described herein can also have multiple moieties such as one or more of ethylene oxide, propylene oxide, ether, ethylene glycol and propylene glycol moieties along with one or more other moieties.

Further, if either or both of the first and second layers are considered to be copolymers, the copolymers can be random copolymers or block copolymers. In some cases, any of the copolymers of the first layer 13 can be block copolymers, for example blocks of a first polymeric unit (e.g., polyester) and blocks of a second polymeric unit (e.g., polyurethane). In some cases, the blocks of the first and/or second polymeric units of the copolymer can have a number average molecular weight from about 3,000 to about 12,000 or from about 3,000 to about 10,000. The finished number average molecular weight of the copolymer can be from about 70,000 to about 110,000 or from about 80,000 to about 100,000 or about 90,000. The copolymer can also be a random copolymer with the above finished number average molecular weights.

In some embodiments, any of the copolymers of the second layer 14 can be block copolymers, for example blocks of a third polymeric unit and (e.g., polyurethane) and blocks of a fourth polymeric unit (e.g., poly (ethylene oxide), poly (propylene oxide), polyether, poly (ethylene glycol) or poly (propylene glycol)). In some cases, the blocks of the third and/or fourth polymeric unit of the copolymer can have a number average molecular weight from about 3,000 to about 12,000 or from about 3,000 to about 10,000. The finished number average molecular weight of the copolymer can be from about 70,000 to about 110,000 or from about 80,000 to about 100,000 or about 90,000. The ratio of the amount (based on molecular weight) of the third polymeric unit to the amount (based on the molecular weight) of the fourth polymeric unit can be between about 40:60 and about 60:40. The copolymer can also be a random copolymer with the above number average molecular weights and ratios of third polymeric units to fourth polymeric units.

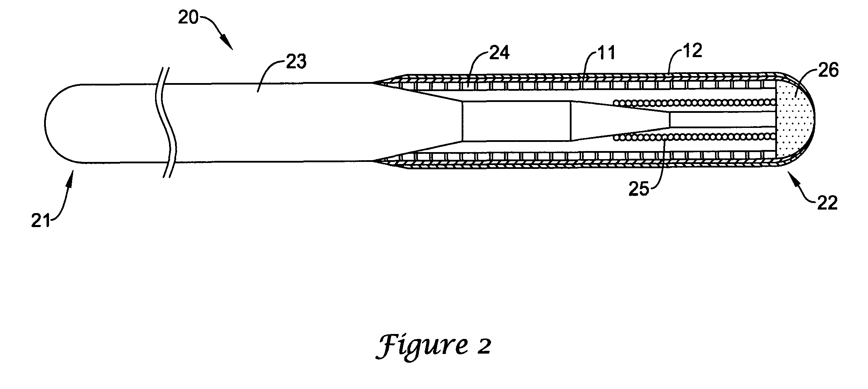

Turning to Figure 2, the coatings described herein can be disposed on a guidewire. The guidewire can have a variety of designs, for example the guidewire can have any of the designs disclosed in U.S. Patent Nos. 6,918,882; or 7,182,735; or U.S. Patent Publication Nos. 2004/0111044; or 2003/0009208. Any of these guidewire designs, or others, can have any of the coatings described herein disposed on a distal portion of the guidewire (which can include the distal end of the guidewire), on a proximal portion of the guidewire (which can include the proximal end of the guidewire), or on an intermediate portion of the guidewire. Further, any of the coatings described herein can be disposed on substantially all, or on the entire, outer exposed surface of any guidewire design.

In Figure 2, a longitudinal cross-sectional view of an example guidewire is shown. In this example the guidewire 20 comprises an elongate core 23 that can be tapered, for example tapered in a distal direction as shown. A coil 25 may be disposed around a portion of, for example the distal portion of, the elongate core 23. Further, a tubular member 24 can be disposed about a portion of the elongate core 23. The elongate core 23 can have a proximal section that is proximal of the tubular member 24 and a distal section that is disposed within the tubular member 24. As can be appreciated, this is but one example embodiment of a guidewire, and a broad variety of other configurations may be used. For example, other structures and/or functional components, such as wires, tubes, marker members, braids, coils, extension members, centering rings, spring tips, polymer tips, or the like, may be included in certain guidewire constructions.

The tubular member 24 can have slots formed in the wall of the tubular member 24 in all or in a portion of the length of the tubular member 24. These slots can enhance the flexibility of all or a portion of the tubular member 24, while allowing for a desired degree of tortioanl rigidity to be maintained. The slots can be formed in a variety of patterns and by a variety of processes. Some example embodiments of appropriate micromachining methods and other cutting methods, and structures for tubular members including slots and medical devices including tubular members are disclosed in U.S. Pat. Publication Nos. US 2003/0069522 and US 2004/0181174-A2; and U.S. Pat. Nos. 6,766,720; and 6,579,246. Some example embodiments of etching processes are described in U.S. Pat. No. 5,106,455.

The elongate core 23 and the tubular member 24 can comprise a variety of materials. For example, the elongate core member 23 or the tubular member 24, or both, can be metallic. The metallic materials can be any of the metallic materials mentioned above with respect to the medical device of Figure 1. In other embodiments, the elongate core 23 and/or the tubular member 24 may comprise a polymer, or one or both of these structures can comprise a ceramic material. The polymer can be any of the materials mentioned above with respect to the medical device of Figure 1.

In one embodiment, the elongate core 23 can comprise stainless steel and the tubular member 24 can also comprise stainless steel or the tubular member 24 can comprise Nitinol, for example any of the Nitinol alloys mentioned herein. Further, it is also contemplated that the elongate core 23 can comprise Nitinol, for example any of the Nitinol alloys mentioned herein; and the tubular member 24 can comprise stainless steel or the tubular member 24 can also comprise Nitinol, for example any of the Nitinol alloys mentioned herein. In addition, either the elongate core 23 or the tubular member 24, or both, could comprise a ceramic material.

Further, a coating 12 can be disposed on at least a portion of the surface of the guidewire 20. The coating 12 can comprise first and second layers. The first layer can comprise a first and second functionalities and/or polymer units, as discussed above, and the second layer can comprise third and fourth functionalities and/or polymer units, as discussed above. For example, the coating can comprise any of the first and second layers disclosed herein. As shown, the coating 12 can be disposed on the surface 11 along the entire length of the tubular member 24. The coating 12 could also extend along only a portion of the tubular member 24, for example a distal portion that can include the distal end of the tubular member 24, a proximal portion that can include the proximal end of the tubular member 24, or an intermediate portion between the distal and proximal ends of the tubular member 24.

The coating 12 can also be disposed on the distal tip 26 in addition to, or in lieu of, any of the above coating configurations on the tubular member 24. For example, the coating 12 can be disposed on a proximal portion of the distal tip 26, as shown in Figure 2. In other embodiments, the coating 12 can be disposed on the entire distal tip 26, or on a distal portion of the distal tip 26.

Further, the coating 12 can be disposed on a portion of or all of the outer surface of the elongate core 23 in addition to, or in lieu of, any of the coatings on the distal tip 26 and/or any of the coatings on the tubular member 24. The coating 12 can be disposed on a distal portion including the distal end of the proximal section of the elongate core 23, as shown in Figure 2. In other embodiments, the coating can be disposed on a proximal portion of the proximal section of the elongate core 23. In some cases, the proximal portion of the proximal section of the elongate core 23 can include the proximal end of the elongate core 23. In other embodiments the coating 12 can be disposed on an intermediate portion of the proximal section of the elongate core 23, for example between the proximal and distal ends of the proximal section of the elongate core 23. In yet another embodiment, the coating 12 can be disposed on substantially entire, or the entire, surface of the proximal section of the elongate member 23. In such a case, the coating 12 can cover the entire, or substantially the entire, outer surface of the guidewire 20.

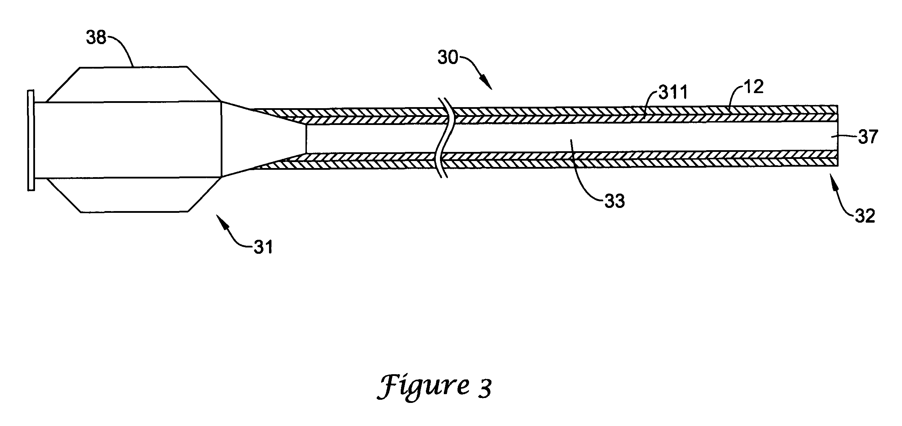

Turning to Figure 3, a perspective view of an example catheter 30 is shown. In addition to the catheter design discussed herein, the catheter can also have other designs and the catheter can comprise a variety of materials, for example the catheter designs and materials disclosed in U.S. Patent Nos. 7,001,369; 5,569,218; 5,603,705; 5,674,208; 5,680,873; 5,733,248; 5,853,400; 5,860,963; and 5,911,715. In some designs, the shaft of the catheter could also have medical devices disposed on it, for example an angioplasty balloon or a stent and stent deployment device or other device.

In the embodiment of Figure 3, the catheter 30 has a shaft 33 with a surface 311. The catheter shaft 33 can comprise any suitable material or any suitable combination of materials. In some embodiments, the shaft 33 can comprise one or more polymeric materials, for example any of the polymeric materials listed above with respect to Figure 1. In other embodiments the shaft 33 can comprise one or more metallic materials, for example any of the metallic materials listed above with respect to Figure 1. In yet other embodiments, the catheter shaft 33 can comprise a combination of polymeric and metallic materials, for example the catheter shaft 33 can have a proximal portion comprising a metallic material (e.g., stainless steel or a Nitinol alloy) and a distal portion comprising a more flexible metallic material (e.g., a more flexible stainless steel or Nitinol structure) or a polymeric material.

The coating 12 can be disposed on substantially the entire, or the entire (as shown in Figure 3), surface 311 of the catheter shaft 33 (the surface 311 being the outer exposed surface). The coating 12 can also be disposed only on a distal portion of the catheter shaft 33, and the distal portion can include the distal end 32 of the shaft 33. In other cases the coating 12 can be disposed only on a proximal portion of the catheter shaft 33, and the proximal portion can include the proximal end 31 of the shaft 33. In other embodiments, the coating 12 can be disposed on an intermediate portion between the proximal and distal ends (31, 32). In yet other embodiments, the coating 12 can be disposed on the inner surface of the catheter shaft lumen 37 in addition to, or in lieu of, any of the above coating configurations on at least a portion of the outside surface 311 of the catheter shaft. The coating 12 of Figure 3 can be any of the coatings that are described herein.

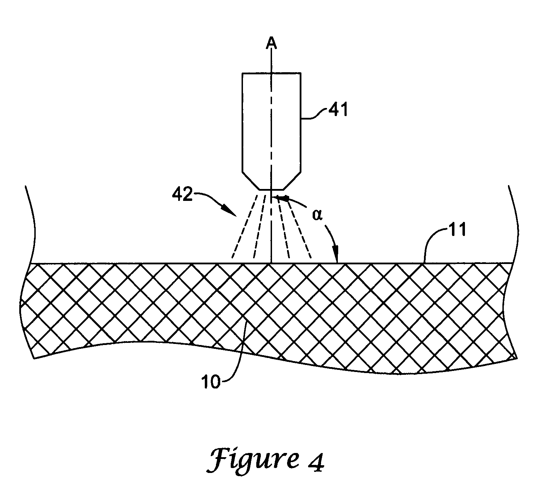

In another example embodiment, a method of coating a medical device is disclosed. Turning to Figure 4, a medical device 10 (which can be any of the medical devices discussed herein or others) having a surface 11 is shown with the surface disposed in a horizontal orientation. It is also contemplated that the surface 11 can be disposed in a vertical orientation facing up, an orientation between a vertical orientation and horizontal orientation facing up, or a partially or entirely inverted orientation. A spray nozzle 41 can be positioned at an angle α of about a 90 degree orientation to the surface 11. The spray nozzle 41 can also be oriented at other angles, for example at about an 80 degree orientation, at about an 85 degree orientation, at about a 95 degree orientation, or at about a 100 degree orientation to the surface 11. The spray nozzle 41 can be used to form a spray pattern 42 of a first coating formulation on the surface 11 of the medical device 10. The first coating formulation can be a formulation of a solvent with components and/or precursors of the first layer 13 (e.g. first and second functionalities, and/or precursors thereof) dissolved and/or suspended and/or dispersed in the solvent. For example, the first layer can be any of the first layers described herein, and the solvent can be water or an organic solvent, or a mixture of water and an organic solvent. In one embodiment, the solvent has between about a 1:4.0 and about a 1:4.2 ratio of 1-methyl-pyrrilidone:deionized water. Further, the first coating formulation can also have other additives that can facilitate handling of the formulation, for example surfactants.

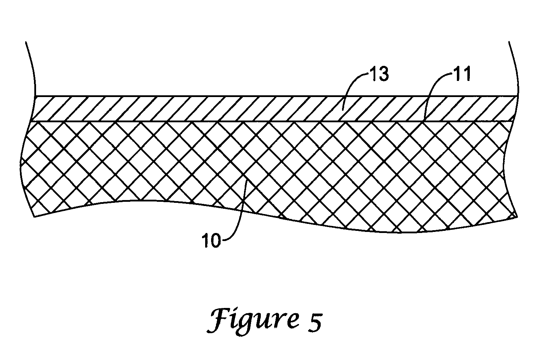

After the first coating formulation containing is sprayed on the surface 10, the formulation can be dried, which can eliminate most or all of the solvent, forming the first layer 13 as shown in Figure 5. In other cases, the applied first coating formulation can remain on the surface 11 with most or all of the solvent present.

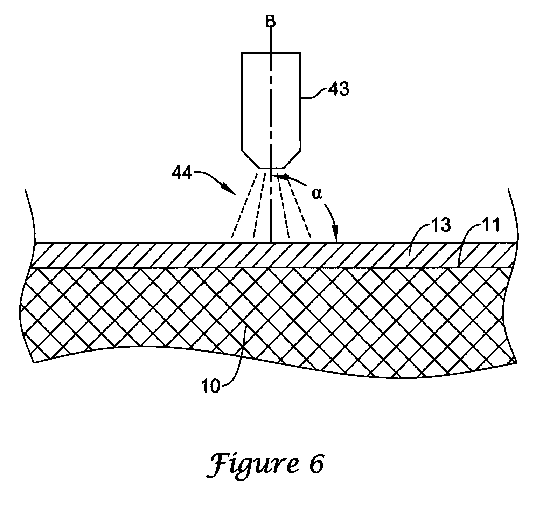

Turning to Figure 6, the same or a different spray nozzle 43 can be used to apply a second coating formulation to form the second layer 14, for example any of the second layers discussed herein. The surface 11 can be disposed in any of the orientations discussed above with respect to Figure 4. Also, the spray nozzle 43 can be disposed in relation to the surface 11 at any of the angles discussed above with respect to Figure 4. The spray nozzle 43 can be used to form a spray pattern 44 of a second coating formulation on the surface 11 of the medical device 10. The second coating formulation can be a formulation of a solvent with components and/or precursors of the second layer (e.g. third and fourth functionalities, and/or precursors thereof) dissolved and/or suspended and/or dispersed in the solvent. For example, the second layer can be any of the second layers described herein, and the solvent can be water or an organic solvent or a mixture of water and an organic solvent. In one embodiment, the solvent is about a 1:4 ratio of water: isopropanol. Further, the second coating formulation can also have other additives that can facilitate handling of the formulation, for example surfactants.

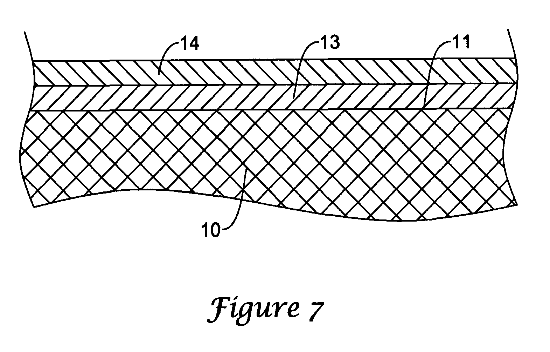

After the second coating formulation is applied, the formulation can be dried, in some cases removing all or a portion of the solvent. In cases where the first formulation was not completely dried before applying the second formulation, the drying process for the second formulation can also evaporate some or all of the remaining solvent from the first formulation. A two layer coating 12 that can result from the above process is shown in Figure 7.

Further, it is also contemplated that the first and second layers (13, 14) can be at least partially mixed at the interface between the layers. In such a case, in at least part of the coating there can be a gradient where the ratio of the first to the second layer changes from all or mostly the first layer to all or mostly all the second layer. The change in composition can occur from an inner portion of the coating relatively closer to the surface of the medical device toward an outer portion of the coating relatively further from the surface of the medical device. As an example, if the first formulation were not completely dried (e.g., not dried at all), then at least part of the second formulation may mix with the first formulation upon application of the second formulation. In such a case, a gradient can be formed where the coating changes from all or mostly all the first layer at a point relatively closer to the surface (11, 311) to all or mostly all the second layer at a point relatively closer to the outer surface of the coating. In some cases, this gradient can exist in the portion of the coating immediately adjacent the interface between the layers, and in other cases the gradient could exist through most, or all, of the thickness of the coating.

Below are some specific examples of coatings:

### EXAMPLE I

In example 1, first and second formulations were applied to a Nitinol surface of slotted tubular members. The first formulation comprised SANCURE 1073C at 12.5% by weight, 0.4% by weight of TRITON X-100 and the remainder was a solvent mixture of 1-methyl-2-pyrrilidone (NMP) and deionized water in a ratio of 1:4. SANCURE 1073C is an aliphatic polyurethane polyester base dispersion including both polyurethane and polyester functionalities and/or polymer units, and is commercially available from Noveon, Inc. This first formulation was applied using a SONO-TEK ultrasonic nozzle (120kHz) at a flow rate of 0.25 mL/min. The second formulation comprised a Tecophilic polyurethane, or TECOGEL, TG-500B from NOVEON, Inc) at 5% by weight in a solvent of isopropanol and deionized water at a ratio of 4:1. The Tecophilic polyurethane comprises a copolymer of a polyurethane and a poly (ethylene oxide), and is commercially available from Noveon, Inc.. The second formulation was applied using an EFD 780 air atomized nozzle.

The tubular members to be coated were ultrasonically cleaned and rinsed. Each of the tubular members was coated with the first formulation. The tubular members were rotated at 60 rpm and were passed twice through the spray pattern of the first formulation at a rate of 0.222 inches/second. The tubular members were then rotated at 60 rpm while being passed once through a spray pattern of the second formulation at 0.666 inches/second and at pressure conditions of 15psi atomization pressure and 5psi pot pressure. The tubular member was then placed within a convection oven at 130 degrees Celsius for 5 minutes.

### EXAMPLE 2

In example 2, tubular members were coated using a process similar to that described with respect to example 1, except that the tubular members were only passed through the spray pattern of the first formulation once.

### EXAMPLE 3

In example 3, tubular members were coated using a process similar to that described with respect to example 1, except that the tubular members were passed once through a spray pattern of the first formulation at a speed of 0.333 inches/second.

### EXAMPLE 4

In example 4, tubular members were coated using a process similar to that described with respect to example 1, except that the tubular members were passed once through a spray pattern of the first formulation at a speed of 0.444 inches/second.

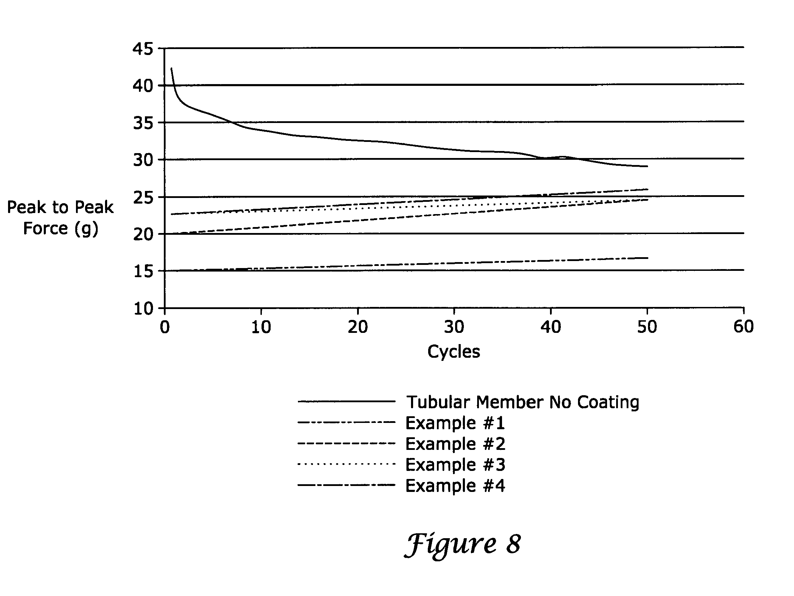

Samples of examples 1-4 with only the first formulation applied were examined using SEM, and example I appeared to have the best coverage of the first formulation across the surface of the tubular member. Significant visual differences were not noticed between examples 2-4. In addition, the finished tubular members were also tested for lubricity and the durability of the lubricity. Figure 8 shows the lubricity over a number of wear cycles for examples 1-4 and for a tubular member without any coating. The graph shows that initial lubricity is higher and that maintaining lubricity is more effective with the better SANCURE 1073C coverage of Example 1. Lubricity was initially approximately 71% better with two coats of SANCURE 1073C versus one coat, and 47% more durable over fifty wear cycles.

### EXAMPLE 5

Example 5 used first and second formulations that were the same as the first and second formulations used in Example 1. The same type of SONO-TEK ultrasonic nozzle (120 kHz) was used for spraying the first formulation nozzle, while a SONO-TEK ultrasonic nozzle (60kHz) was also used for spraying the second formulation in place of the EFD 780 nozzle of Example 1. One pass of the first formulation was applied at a 0.22 inches per second speed for the tubular member, a 0.25 mL/min flow rate through the nozzle, 28 psi delivery pressure, and the first formulation was dried at 130 degrees Celsius for 2 minutes before the second formulation was applied. After application of the second formulation, the tubular member was dried for 5 minutes at 130 degrees Celsius. This example coating yielded a lubricity of 15.73 grams and a durability range of 1.81 grams.

## Claims

1. A medical device having an elongated portion having a surface with a coating disposed on at least a portion of the surface, the coating comprising:
a first layer disposed on at least a portion of the surface of the medical device, the first layer comprising a first polymeric unit and a second polymeric unit, the first polymeric unit including a polyester and adhering to the surface and the second polymeric unit comprising polyurethane; and
a second layer disposed on at least a portion of the first layer, the second layer comprising a third polymeric unit and a fourth polymeric unit, the third polymeric unit adhering to the second polyurethane polymeric unit and the fourth polymeric unit being hydrophilic; and
wherein the interface between the first and second layers is free of covalent bonding between the first and second layers.

2. The medical device of claim 1, wherein the second layer comprises a copolymer of the third polymeric unit and the fourth polymeric unit or a dispersion of the third polymeric unit and the fourth polymeric unit.

3. The medical device of claims 1 or 2, wherein the first layer comprises a dispersion of the first polymeric unit and the second polymeric unit or a copolymer of the first polymeric unit and the second polymeric unit.

4. The medical device of claims 1, 2, or 3, wherein the third polymeric unit is the same polymeric unit as the second polymeric unit.

5. The medical device of claims 1, 2, or 3, wherein the second and third polyurethane polymeric units are different.

6. The medical device of claims 1, 2, 3, 4, or 5, wherein the medical device is a guidewire or a catheter.

7. The medical device of claims 1, 2, 3, 4, 5 or 6, wherein the fourth polymeric unit is a polymeric unit selected from the group consisting of poly (ethylene oxide), poly (propylene oxide), polyether, poly (ethylene glycol), and poly (propylene glycol).

8. The medical device of claims 1, 2, 3, 4, 5, 6 or 7, wherein the surface is a metallic surface.

9. The medical device of claim 8, wherein the surface is a nickel-titanium alloy or stainless steel surface.

10. The medical device of claims 1, 2, 3, 4, 5, 6, 7, 8 or 9, wherein the fourth polymeric unit is a hydrogel.

11. The medical device of claim 1, wherein the fourth polymeric unit is a hydrogel and the hydrogel can absorb five times or more the weight of the hydrogel in water.

12. The medical device of any of claims 1-11, wherein the first layer consists of the first polymeric unit and the second polymeric unit.

13. The medical device of any of claims 1-11, wherein the second layer consists of the third polymeric unit and the fourth polymeric unit.

14. A method of applying a coating to a medical device, the method comprising:
providing a medical device comprising an elongated member having a surface;
applying a first layer of the coating to at least a portion of the surface, the first layer comprising a first polymeric unit and a second polymeric unit, the first polymeric unit including a polyester and adhering to the surface and the second polymeric unit comprising polyurethane; and
applying a second layer of the coating to at least a portion of the first layer, the second layer comprising a third polymeric unit and a fourth polymeric unit, the third polymeric unit comprising polyurethane and the fourth polymeric unit having hydrophilic properties; and
wherein the interface between the first and second layers is free of covalent bonding between the first and second layers.

15. The method of claim 14, wherein the second layer comprises a copolymer of the third polymeric unit and the fourth polymeric unit.

16. The method of claims 14 or 15, wherein the first layer comprises a dispersion of the first polymeric unit and the second polymeric unit.

17. The method of any of claims 14-16, wherein applying the first layer or the second layer includes spray coating the first layer or the second layer.

18. The method of any of claims 14-17, wherein the second layer is applied in a second formulation comprising a solvent and components of the second layer, and, after applying the second formulation, the solvent is dried, forming the second layer.

19. The method of claim 18, wherein the solvent comprises isopropanol and water.

20. The method of any of claims 14-19, wherein the interface between the first layer and the surface of the medical device is free of covalent bonding between the first layer and the medical device.

21. The method of any of claims 14-20, wherein the second layer is a copolymer of polyurethane and a hydrophilic polymer selected from the group consisting of poly (ethylene oxide), poly (propylene oxide), polyether, poly (ethylene glycol) and poly (propylene glycol).

22. The method of any of claims 14-20, wherein the fourth polymeric unit is a hydrogel.

23. The method of claim 22, wherein the hydrogel can absorb five times or more the weight of the hydrogel in water.

## Patentansprüche

1. Eine Medizinprodukt mit einem langgestreckten Teilbereich, welches eine Oberfläche mit einer Beschichtung aufweist, die auf mindestens einem Teil der Oberfläche aufgebracht ist, wobei die Beschichtung umfasst:
eine erste Schicht, die auf mindestens einem Teilbereich der Oberfläche des Medizinproduktes aufgebracht ist, wobei die erste Schicht eine erste Polymereinheit und eine zweite Polymereinheit umfasst, wobei die erste Polymereinheit einen Polyester enthält und an der Oberfläche anhaftet und die zweite Polymereinheit Polyurethan umfasst; und
eine zweite Schicht, die auf mindestens einem Teilbereich der ersten Schicht aufgebracht ist, wobei die zweite Schicht eine dritte Polymereinheit und eine vierte Polymereinheit umfasst, wobei die dritte Polymereinheit an der zweiten Polyurethanpolymereinheit anhaftet und die vierte Polymereinheit hydrophil ist; und
wobei die Grenzfläche zwischen den ersten und zweiten Schichten frei von kovalenten Bindungen zwischen der ersten und zweiten Schicht ist.

2. Das Medizinprodukt nach Anspruch 1, wobei die zweite Schicht ein Copolymer aus der dritten Polymereinheit und der vierten Polymereinheit oder eine Dispersion aus der dritten Polymereinheit und der vierten Polymereinheit umfasst.

3. Das Medizinprodukt nach Anspruch 1 oder 2, wobei die erste Schicht eine Dispersion aus der ersten Polymereinheit und der zweiten Polymereinheit oder ein Copolymer aus der ersten Polymereinheit und der zweiten Polymereinheit umfasst.

4. Das Medizinprodukt nach den Ansprüchen 1, 2 oder 3, wobei die dritte Polymereinheit die gleiche Polymereinheit ist wie die zweite Polymereinheit.

5. Das Medizinprodukt nach den Ansprüchen 1, 2 oder 3, wobei die zweite Polymereinheit und die dritte Polymereinheit unterschiedlich sind.

6. Das Medizinprodukt nach den Ansprüchen 1, 2, 3, 4 oder 5, wobei das Medizinprodukt ein Führungsdraht oder ein Katheter ist.

7. Das Medizinprodukt nach den Ansprüchen 1, 2, 3, 4, 5 oder 6, wobei die vierte Polymereinheit eine Polymereinheit, ausgewählt aus der Gruppe bestehend aus Poly(ethylenoxid), Poly(propylenoxid), Polyether, Poly(ethylenglykol) und Poly(propylenglykol), ist.

8. Das Medizinprodukt nach den Ansprüchen 1, 2, 3, 4, 5, 6 oder 7, wobei die Oberfläche eine metallische Oberfläche ist.

9. Das Medizinprodukt nach Anspruch 8, wobei die Oberfläche eine Oberfläche aus Nickel-Titan-Legierung oder eine Oberfläche aus Edelstahl ist.

10. Das Medizinprodukt nach den Ansprüchen 1, 2, 3, 4, 5, 6, 7, 8 oder 9, wobei die vierte Polymereinheit ein Hydrogel ist.

11. Das Medizinprodukt nach Anspruch 1, wobei die vierte Polymereinheit ein Hydrogel ist und das Hydrogel das Fünffache oder mehr des Gewichts des Hydrogels an Wasser absorbieren kann.

12. Das Medizinprodukt nach einem der Ansprüche 1-11, wobei die erste Schicht aus der ersten Polymereinheit und der zweiten Polymereinheit besteht.

13. Das Medizinprodukt nach einem der Ansprüche 1-11, wobei die zweite Schicht aus der dritten Polymereinheit und der vierten Polymereinheit besteht.

14. Ein Verfahren zum Aufbringen einer Beschichtung auf ein Medizinprodukt, wobei das Verfahren umfasst:
Bereitstellen eines Medizinproduktes, welches ein langgestrecktes Element mit einer Oberfläche umfasst;
Aufbringen einer ersten Schicht der Beschichtung auf mindestens einen Teilbereich der Oberfläche, wobei die erste Schicht eine erste Polymereinheit und eine zweite Polymereinheit umfasst, wobei die erste Polymereinheit einen Polyester enthält und an der Oberfläche anhaftet und die zweite Polymereinheit Polyurethan umfasst; und
Aufbringen einer zweiten Schicht der Beschichtung auf mindestens einen Teilbereich der ersten Schicht, wobei die zweite Schicht eine dritte Polymereinheit und eine vierte Polymereinheit umfasst, wobei die dritte Polymereinheit Polyurethan umfasst und die vierte Polymereinheit hydrophile Eigenschaften aufweist; und
wobei die Grenzfläche zwischen den ersten und zweiten Schichten frei von kovalenten Bindungen zwischen der ersten und zweiten Schicht ist.

15. Das Verfahren nach Anspruch 14, wobei die zweite Schicht ein Copolymer aus der dritten Polymereinheit und der vierten Polymereinheit umfasst.

16. Das Verfahren nach den Ansprüchen 14 oder 15, wobei die erste Schicht eine Dispersion aus der ersten Polymereinheit und der zweiten Polymereinheit umfasst.

17. Das Verfahren nach einem der Ansprüche 14-16, wobei das Aufbringen der ersten Schicht oder der zweiten Schicht Sprühbeschichten der ersten Schicht oder der zweiten Schicht einschließt.

18. Das Verfahren nach einem der Ansprüche 14-17, wobei die zweite Schicht in einer zweiten Zubereitung, umfassend ein Lösungsmittel und Bestandteile der zweiten Schicht, aufgebracht wird, und, nach dem Auftragen der zweiten Zubereitung, das Lösungsmittel getrocknet wird, um die zweite Schicht zu bilden.

19. Das Verfahren nach Anspruch 18, wobei das Lösungsmittel Isopropanol und Wasser umfasst.

20. Das Verfahren nach einem der Ansprüche 14-19, wobei die Grenzfläche zwischen der ersten Schicht und der Oberfläche des Medizinproduktes frei von kovalenten Bindungen zwischen der ersten Schicht und dem Medizinprodukt ist.

21. Das Verfahren nach einem der Ansprüche 14-20, wobei die zweite Schicht ein Copolymer aus Polyurethan und einem hydrophilen Polymer, ausgewählt aus der Gruppe bestehend aus Poly(ethylenoxid), Poly(propylenoxid), Polyether, Poly(ethylenglykol) und Poly(propylenglykol), ist.

22. Das Verfahren nach einem der Ansprüche 14-20, wobei die vierte Polymereinheit ein Hydrogel ist.

23. Das Verfahren nach Anspruch 22, wobei das Hydrogel das Fünffache oder mehr des Gewichts des Hydrogels an Wasser absorbieren kann.

## Revendications

1. Dispositif médical présentant une portion allongée ayant une surface avec un revêtement disposé sur au moins une portion de la surface, le revêtement comprenant :
une première couche disposée sur au moins une portion de la surface du dispositif médical, la première couche comprenant une première unité polymère et une seconde unité polymère, la première unité polymère comprenant un polyester et adhérant à la surface et la seconde unité polymère comprenant du polyuréthane ; et
une seconde couche disposée sur au moins une portion de la première couche, la seconde couche comprenant une troisième unité polymère et une quatrième unité polymère, la troisième unité polymère adhérant à la seconde unité polymère de polyuréthane et la quatrième unité polymère étant hydrophile ; et
dans lequel l'interface entre les première et seconde couches est exempte de liaison covalente entre les première et seconde couches.

2. Dispositif médical selon la revendication 1, dans lequel la seconde couche comprend un copolymère de la troisième unité polymère et de la quatrième unité polymère ou une dispersion de la troisième unité polymère et de la quatrième unité polymère.

3. Dispositif médical selon la revendication 1 ou 2, dans lequel la première couche comprend une dispersion de la première unité polymère et de la seconde unité polymère ou un copolymère de la première unité polymère et de la seconde unité polymère.

4. Dispositif médical selon les revendications 1, 2, ou 3, dans lequel la troisième unité polymère est la même unité polymère que la seconde unité polymère.

5. Dispositif médical selon les revendications 1, 2, ou 3, dans lequel les seconde et troisième unités polymères de polyuréthane sont différentes.

6. Dispositif médical selon les revendications 1, 2, 3, 4, ou 5, dans lequel le dispositif médical est un câble de guidage ou un cathéter.

7. Dispositif médical selon les revendications 1, 2, 3, 4, 5, ou 6, dans lequel la quatrième unité polymère est une unité polymère choisie dans le groupe constitué de poly(oxyde d'éthylène), de poly(oxyde de propylène), de polyéther, de poly(éthylène glycol), et de poly(propylène glycol).

8. Dispositif médical selon les revendications 1, 2, 3, 4, 5, 6 ou 7, dans lequel la surface est une surface métallique.

9. Dispositif médical selon la revendication 8, dans lequel la surface est une surface d'alliage de nickel-titane ou d'acier inoxydable.

10. Dispositif médical selon les revendications 1, 2, 3, 4, 5, 6, 7, 8 ou 9, dans lequel la quatrième unité polymère est un hydrogel.

11. Dispositif médical selon la revendication 1, dans lequel la quatrième unité polymère est un hydrogel et l'hydrogel peut absorber cinq fois ou plus la masse de l'hydrogel dans l'eau.

12. Dispositif médical selon l'une quelconque des revendications 1-11, dans lequel la première couche est constituée de la première unité polymère et de la seconde unité polymère.

13. Dispositif médical selon l'une quelconque des revendications 1-11, dans lequel la seconde couche est constituée de la troisième unité polymère et de la quatrième unité polymère.

14. Procédé d'application d'un revêtement sur un dispositif médical, le procédé comprenant les étapes consistant :
à fournir un dispositif médical comprenant un élément allongé présentant une surface ;
à appliquer une première couche du revêtement sur au moins une portion de la surface, la première couche comprenant une première unité polymère et une seconde unité polymère, la première unité polymère comprenant un polyester et adhérant à la surface et la seconde unité polymère comprenant du polyuréthane ; et
à appliquer une seconde couche du revêtement sur au moins une portion de la première couche, la seconde couche comprenant une troisième unité polymère et une quatrième unité polymère, la troisième unité polymère comprenant du polyuréthane et la quatrième unité polymère présentant des propriétés hydrophiles ; et
dans lequel l'interface entre les première et seconde couches est exempt de liaison covalente entre les première et seconde couches.

15. Procédé selon la revendication 14, dans lequel la seconde couche comprend un copolymère de la troisième unité polymère et de la quatrième unité polymère.

16. Procédé selon les revendications 14 ou 15, dans lequel la première couche comprend une dispersion de la première unité polymère et de la seconde unité polymère.

17. Procédé selon l'une quelconque des revendications 14-16, dans lequel l'application de la première couche ou de la seconde couche comprend un revêtement par pulvérisation de la première couche ou de la seconde couche.

18. Procédé selon l'une quelconque des revendications 14-17, dans lequel la seconde couche est appliquée dans une seconde formulation comprenant un solvant et des constituants de la seconde couche, et, après l'application de la seconde formulation, le solvant est séché, formant la seconde couche.

19. Procédé selon la revendication 18, dans lequel le solvant comprend de l'isopropanol et de l'eau.

20. Procédé selon l'une quelconque des revendications 14-19, dans lequel l'interface entre la première couche et la surface du dispositif médical est exempte de liaison covalente entre la première couche et le dispositif médical.

21. Procédé selon l'une quelconque des revendications 14-20, dans lequel la seconde couche est un copolymère de polyuréthane et un polymère hydrophile choisi dans le groupe constitué de poly(oxyde d'éthylène), de poly(oxyde de propylène), de polyéther, de poly(éthylène glycol), et de poly(propylène glycol).

22. Procédé selon l'une quelconque des revendications 14-20, dans lequel la quatrième unité polymère est un hydrogel.

23. Procédé selon la revendication 22, dans lequel l'hydrogel peut absorber cinq fois ou plus la masse de l'hydrogel dans de l'eau.
